# EUROPEAN PATENT SPECIFICATION

(11) **EP 0 661 965 B1**
(45) Date of publication and mention of the grant of the patent: **30.06.1999**
(21) Application number: 93921662.8
(22) Date of filing: 22.09.1993
(51) Int. Cl.: A61K 7/50, A61K 7/06

(54) **HAIR CONDITIONING STYLE CONTROL SHAMPOO**
HAARPFLEGE- UND FIXIERUNGSMITTEL
SHAMPOOING CAPILLAIRE TRAITANT ET COIFFANT

(30) Priority: 22.09.1992 US 948516; 13.09.1993 US 118411
(43) Date of publication of application: 12.07.1995
(73) Proprietor: Colgate-Palmolive Company, New York, N.Y. 10022-7499 (US)
(72) Inventor: PATEL, Amrit, Dayton, NJ 08810 (US); ROBBINS, Clarence R., Martinsville, NJ 08836 (US)
(74) Representative: UEXKÜLL & STOLBERG
(86) International application number: US9308822
(87) International publication number: WO9406410

(56) References cited:
- EP-A- 0 413 417
- WO-A-92/10162
- GB-A- 2 193 971

## Description

The present invention relates to shampoo compositions capable of cleansing the hair, as well as providing hair conditioning and style control.

### Discussion of the Prior Art

Numerous attempts have been made to provide so-called three-in-one shampoo compositions, i.e., shampoo formulations which will provide not only hair cleansing action, but also hair conditioning effects and style control or set and curl retention of the hair following completion of the shampooing and rinsing operations.

Hair conditioning effects are usually described as softness and smoothness of the hair after washing, a less sticky feeling, easy passage of the comb through the hair, a less hard or rough feeling after shampooing, relief from static electricity or "fly-away" and manageability.

Hair styling, i.e., hair style retention or hold or set and curl retention can be accomplished in two ways. First, the hair can be subjected to a permanent chemical alteration to achieve a certain desired shape. Secondly, a temporary alteration of the shape of the hair can be achieved, generally by applying to the hair a third composition following shampooing and/or hair conditioning agent. The materials comprising these temporary style control compositions have traditionally been resins, gums, etc., and are usually applied to the previously shampooed and/or conditioned hair in the form of mousses, gels, lotions or sprays. Drying the thus-treated hair results in a "set" of the resinous or gum material to a stiff condition which temporarily holds the hair in the desired shape.

The temporary style control agents differ from the permanent style control treatments in that the latter are marginally affected by subsequent shampooing operations, whereas the temporary agents are completely removed upon shampooing thereby requiring their re-application after every shampoo operation in order to maintain the desired style control.

One type of temporary style control agent are the adhesive polymers which generally comprise a non-water solvent, and a soluble rigid polymer having a glass transition temperature above the temperature normally reached in styling operations, i.e., blow drying, etc. Examples of such polymers are those described in U.S. Patent Nos. 3,743,715, 4,165,367 and 4,223,039.

Other polymers heretofore suggested for temporary style control are multi-component polymers which combine up to four or more monomers. Exemplary of these types of style control polymer systems are those disclosed in U.S. Patent Nos. 3,222,329, 3,577,517, 4,012,501 and 4,272,511.

Block copolymers having multiple glass transition temperatures have also been employed as style control agents, such as those described in U.S. Patent Nos. 3,907,984, 4,030,512 and 4,283,384.

U.S. Patent No. 5,104,642 describes the use in a hair styling composition of a polymer comprising up to 50% of a first polymerizable hydrophilic monomer and the remainder a second polymerizable hydrophilic monomer, the resulting polymer having a molecular weight of 5,000 to 1,000,000, a glass transition temperature, Tg, of greater than -20°C and a solubility parameter, d, of 8.5 to 12.0. International Application No. PCT/US91/02170, published October 17, 1991, discloses the incorporation of these types of polymers in a hair shampoo formulation.

Canadian Patent No. 2,033,626 describes a shampoo composition which provides set and curl retention comprising at least one anionic surfactant, a film-forming polymer (e.g., Polymer JR, GAFQUAT 755, MERQUAT 550, chitosan or its derivatives, CROQUAT S, LAMEQUAT L or methylvinyl imidazolium chloride/vinyl pyrrolidone copolymer and large amounts of citric acid to enhance the effect of the film-forming polymer. There is no disclosure in the patent that hair conditioning properties are possessed by the shampoo formulation.

A major disadvantage of these so-called style control shampoos is that they require the inclusion in the shampoo formulation of a non-water solvent, which is usually a volatile organic compound, in order to solubilize the polymer which is usually non-water soluble. This increases the overall cost of the shampoo composition and provides for a potential environmental hazard. It also contributes to the instability of the system since, if the amount of non-water solvent decreases to below that required to solubilize the polymer, the latter will precipitate out of the composition.

Moreover, for the most part, the style control shampoos heretofore introduced provide no hair conditioning effects unless a third ingredient (in addition to the detergent and style control polymer) is added to the composition. Adding a third type of chemical agent to the already complex typical style control shampoo to achieve hair conditioning further contributes to the cost and difficulty in making the final composition.

One class of hair conditioning agents which has recently found favor in the industry is the so-called cationic polymers, e.g., quaternized cellulose derivatives, quaternized guar gum, quaternary ammonium polymers such as copolymers of certain dialkyldiallyl ammonium halides with, e.g., acrylamide, acrylic acid, etc. For example, U.S. Patent No. 3,996,146 describes a clear hair conditioning shampoo formulation comprising a polymer derived from dimethyldiallyl ammonium salts, one or more of a variety of anionic surfactants, at least one amphoteric detergent and sufficient acid to render the pH of the formulation between 4.0 and 6.7. There is no disclosure in the patent that the described shampoo provides style retention.

Cationic polymers carry a rather high positive charge which renders their inclusion in shampoos which normally contain anionic detergents problematical because of the tendency of the cationic polymers to form complexes with the anionic detergents and thus are difficult to stabilize.

Further, WO 92/10162 discloses a hair conditioning shampoo comprising a cationic polymer having a charge density smaller than 200 and two anionic surfactants, one being long chained non-aromatic and the other being aromatic or short chained.

It is an object of the present invention to provide a hair conditioning, style control shampoo composition which has a high degree of hair conditioning effect and imparts style control to the hair after the shampooing operation, including rinsing, which is not subject to the above-noted disadvantages.

### SUMMARY OF THE INVENTION

The above and other objects are realized by the present invention, one embodiment of which relates to a hair conditioning style control shampoo in aqueous emulsion or suspension form comprising:
a. from 0.1% to 5% of at least one cationic polymer having a hair conditioning effect and a charge density greater than about 200;
b. from 3% to 30% of a first long chain non-aromatic anionic surfactant, wherein said non-aromatic surfactant is selected from the group consisting of alkyl sulfates, alkyl ether sulfates, α-olefin sulfonates, paraffin sulfonates, ethoxylated and propoxylated alkyl ether sulfates containing 1 to 10 ethylene oxide or propylene oxide units per molecule and mixtures thereof, wherein said alkyl groups of said sulfates contain 10 to 18 carbon atoms and may be unsaturated and wherein said alkyl groups of said sulfonates contain 12 to 18 carbon atoms and may be unsaturated;
c. from 0.1% to 10% of at least one second anionic surfactact selected from the group consisting of aromatic sulfonates containing fewer than 8 alkyl carbon atoms, alkyl or alkyl ether sulfates containing up to 8 carbon atoms in the alkyl chain, alkyl sulfonates containing up to 10 carbon atoms in the alkyl group and mixtures thereof; and
d. the remainder water.

A further embodiment of the invention relates to a hair conditioning style control shampoo as described above additionally containing a dispersing agent to stabilize the emulsion or suspension.

A third embodiment of the invention relates to a method for cleansing, conditioning and providing style control to hair with a single composition comprising shampooing the hair with a composition as described above.

### DETAILED DESCRIPTION OF THE INVENTION

The invention is predicated on the discovery that the cationic polymers described above are capable of functioning as both hair conditioning agents and style control agents provided that the shampoo formulation in which they are incorporated contains two separate and well-defined types of anionic surfactants and, further, provided that the cationic polymer possesses a charge density greater than about 200.

The charge density of a cationic polymer is given by the formula cation molecular weight/the number of positive charges. By this definition, a ratio that is lower numerically represents a higher charge density. Thus, the "charge density" of a cationic polymer is a numerical value representing the number of positive charges in a particular cationic polymer. The greater the number of positive charges in a cationic polymer of fixed cation molecular weight, the greater will be the "charge density" of the polymer. However, since the charge density, as measured by the above ratio, is inversely proportional to the number of positive charges relative to the cation molecular weight, the greater the number of positive charges and hence the greater the charge density, the lower the numerical value of the above ratio. Thus, for example, a cationic polymer whose charge density as given by the above ratio is 150 will have a "greater" charge density (i.e., greater number of positive charges per unit of cation molecular weight) than a polymer having a measured "charge density" of 200.

Any cationic polymer having hair conditioning properties and a charge density greater than about 200 may be employed in the practice of the invention, although the preferred charge density is greater than about 130. It will be understood by those skilled in the art that any cationic polymer having a hair conditioning effect which can be crafted having a charge density greater than about 130 may be employed in the practice of the invention. Exemplary of such cationic polymers are dialkyldiallyl ammonium salt (e.g., halide) homopolymers or copolymers, e.g., dimethyldiallyl ammonium chloride homopolymer, dimethyldiallyl ammonium chloride/acrylamide copolymer containing at least 60% dimethyldiallyl ammonium chloride monomer, dimethyldiallyl ammonium chloride/acrylic acid containing at least 90% dimethyldiallyl ammonium chloride monomer, vinyl imidazole/vinyl pyrrolidone copolymers containing at least 50% vinyl imidazole and polyethyleneimine. Currently, the preferred cationic polymers are Merquat 100 [a polymer of diallyldimethyl ammonium chloride (charge density of 126)] and Luviquat 905 [a 95% vinyl imidazole/5% vinylpyrrolidone copolymer (charge density of 116)]. The registered trademarks as used in the following are acknowledged as such.

In some embodiments of the invention, it will be desirable to include in the shampoo formulation small amounts of at least one carbohydrate-containing cationic polymer such as, e.g., cationic guar, and quaternary nitrogen-containing cellulose ether, e.g., Polymer JR or LR, manufactured and sold by Amerchol AS, UCARE Polymers, CELQUAT SC-240 by National Starch, etc. The carbohydrate-containing cationic polymer operates to stabilize the system and enables the inclusion of additional quantities of the high charge density cationic polymer, and thus provides for enhanced style control and conditioning.

The cationic polymer should be present in the shampoo composition in an amount of from about 0.1% to about 5%, and preferably from about 0.25% to about 2.5%.

The first of the anionic surfactants employed in the shampoo of the invention comprises any of the well known, high foaming, high detergency, relatively long chain non-aromatic anionic surfactants conventionally employed in shampoo formulations such as an alkyl sulfate, an alkyl ether sulfate, an alpha-olefin sulfonate, a paraffin sulfonate, an ethoxylated or propoxylated alkyl ether sulfate containing 1 to 10 ethylene oxide or propylene oxide units per molecule, wherein the alkyl groups contain 10 to 18 carbon atoms and may be unsaturated, a sulfosuccinate and mixtures thereof. By relatively long chain is meant an alkyl sulfate or alkyl ether sulfate wherein the carbon chain length of the alkyl group is a minimum of 10 carbon atoms or an alkyl sulfonate wherein the minimum carbon chain length of the alkyl group is 12 carbon atoms. Any of the anionic detergents disclosed in U.S. Patent Nos. 5,106,613 and 4,997,641, may be utilized in the shampoo compositions of the invention provided that the minimum chain lengths are in accordance with the foregoing. Other suitable anionic detergents are described in McCutcheon's Detergents and Emulsifiers, North American ed., published 1984,

The non-aromatic anionic surfactant should be present in the shampoo composition of the invention in an amount of from about 3% to about 30%, and preferably in an amount of from about 10% to about 20%.

The primary purpose of the non-aromatic anionic surfactant, of course, is to provide the necessary detergency to cleanse the hair and scalp. It also functions to disperse the cationic-anionic complex and to improve the style control of the system. In general, maximum style control is obtained using alpha-olefin sulfonate, followed by lauryl sulfate, lauryl ether sulfate and decyl ether sulfate providing the least style control when all other variables are held constant.

The second anionic surfactant employed in the shampoo of the invention is an aromatic or short-chained anionic surfactant. By short-chained is meant an alkyl sulfate or alkyl ether sulfate having a chain length of no more than 8 carbon atoms or an alkyl sulfonate having a chain length no longer than 10 carbon atoms. The presence of the aromatic or short-chained anionic surfactant is essential to achieve significant adhesion of the cationic polymer to the hair. While not wishing to be bound by any theory as to the mechanism of function of the composition of the invention, it appears that the aromatic anionic surfactant combines with the cationic polymer to provide a unique anionic/cationic complex which strongly adheres to the hair fibers and provides strong hair conditioning as well as style control effects.

In addition, it is believed that the reason that only short-chained aromatic species like sodium toluene sulfonate are useful, but homologous long chain length aromatic species like sodium dodecyl benzene sulfonate do not function as the second anionic surfactants and short-chained alkyl sulfates like sodium hexyl sulfate are effective, but related ingredients like sodium deceth-2-sulfate is ineffective as the second anionic surfactant is that longer chain species over-plasticize the cationic-anionic complex and thus inhibit style control, whereas the shorter chain length species somehow in an unexpected manner create an adhesive film from the high charge density cationic in the presence of the primary anionic detergent.

Suitable aromatic anionic surfactants include cumene sulfonate, xylene sulfonate, benzene sulfonate, toluene sulfonate, 1,3-benzene disulfonic acid disodium salt, 1 ,2-benzene disulfonic acid disodium salt, but not dodecyl benzene sulfonate. Suitable short chain length aliphatic ingredients include hexylsulfate, octylsulfate, octyl sulfonate and decyl sulfonate, but not deceth-2-sulfate. Mixtures of the above suitable aromatic and aliphatic second anionic surfactants are also effective.

The aromatic anionic surfactant should be present in the shampoo compositions of the invention in an amount from about 0.1% to about 10%, and preferably from about 1% to about 5%.

In several embodiments of the invention, it is desirable to include in the shampoo formulation one or more amphoteric surfactants which aid in the solubilization of the components formed upon mixing the various ingredients of the shampoo composition such as, e.g., amphopropionic acid, cocamidopropyl betaine, cocobetaine, cocobetainamido sodium lauriminodipropionate, dodecyl dimethylamine oxide, octyl dimethylamine oxide, octadecyl dimethylamine oxide, cocamidopropylamine, cocoamphodipropionic acid, cocamidopropylhydroxy sultaine.

Although the shampoo compositions of the present invention provide adequate hair conditioning activity when employed in most applications, there may be instances where extra conditioning effects are required, for example, in cases of damaged, heavily dyed, bleached, permanent waved or chemically relaxed or straightened hair. For these types of applications, it may be desirable to incorporate a small amount of conventional water-insoluble hair conditioning agents in the composition of the invention to augment the effects of the cationic polymer.

It will be understood by those skilled in the art, however, that the amounts of such water-insoluble hair conditioning agents required in these special cases is lower than the amounts present in commercially available conditioning shampoos to achieve a degree of conditioning equal thereto.

Suitable water-insoluble hair conditioning agents for use in the hair conditioning shampoos of the present invention include silicones, with polydimethylsiloxane (CTFA designation: Dimethicone) a non-volatile silicone fluid, being especially preferred, aminosilicones, polyalkylenes and oxidized derivatives thereof, paraffins, petrolatums, microcrystalline waxes, and C_{18-36 (mixed)} fatty acids and triglycerides and mixtures thereof.

Amounts of water-insoluble hair conditioning agents up to about 5%, and preferably up to about 0.1%, may be utilized in the compositions of the present invention.

It is also preferred to utilize certain dispersing agents to stabilize the emulsions or suspensions which comprise the shampoo compositions of the invention. Suitable dispersing agents include long chain saturated primary aliphatic alcohols or derivatives thereof having an average of 24-45 carbon atoms in the chain; long chain acylated compounds, e.g., esters, acids or amides with at least 18 carbon atoms such as C_{18-36 (mixed)} fatty acids and triglycerides, (CTFA designation: "C18-36 Acid Triglyceride" available as "Syncrowax HGL-C" manufactured by Croda), beeswax, etc.; cross-linked anionic synthetic polymer, e.g., polyacrylic acid or polymethacrylic acid polymers or polysulfonates such as polyvinylsulfate or polystyrene sulfonate, etc.; a polysaccharide or a quaternized derivative thereof such as hydroxyethylcellulose or methylcellulose, guar gum, xanthan gum or quaternized derivatives of the above such as Polymer JR or cationic guar gum; and alkyl dimethyl amine oxide, the alkyl group having from about 10 to about 18 carbon atoms.

Preferably, the dispersing agent is selected so as to render the emulsion or suspension pearlescent.

To make the compositions of the present invention, the various required components are dissolved and/or suspended in an aqueous medium. Such medium may include various non-interfering normal shampoo composition constituents or adjuvants known in the art, but a few of these will be specifically mentioned herein because they are especially desirable components of the present compositions and contribute in a significant manner to its desirable properties. Higher fatty alkanolamides having long been known as foaming agents and foam stabilizers. Such compounds will usually be of 12 to 16 carbon atoms in the acyl group which is reacted with a lower (1 to 3 carbon atoms) mono- or dialkanolamine. In the present formulations, the best alkanolamides are considered to be lauric monoethanolamide and cocodiethanolamide. However, other known foam stabilizers and foaming agents may also be employed in whole or in part such as the betaines and related materials. Various gums and other thickening materials are also useful in shampoo compositions, but it has been found that the best of these in the present compositions are hydroxyethyl celluloses. Such materials are available from Aqualon Corporation under the trademark NATROSOL, such as NATROSOL 250 HHR and NATROSOL 330 CS, which preferably are employed in mixture, with the content of the former being from two to five times that of the latter. However, other suitable gums and thickeners may be also be employed such as hydroxypropylmethyl cellulose, methyl cellulose, modified starches and guar gum. Another important constituent of the present composition is mineral oil when polyethylene is employed as a hair conditioning agent. The mineral oil is employed to solubilize and help disperse the polyethylene which, if not satisfactorily dispersed in the composition, will be of little hair conditioning effect and tends to settle out.

Other components of the present compositions which may be employed include ethylene glycol monostearate, ethylene glycol distearate and propylene glycol distearate, all of which have pearlescing properties; viscosity control agents such as propylene glycol and sodium chloride; pH adjusting agents such as citric acid and citrates; sequestrants such as EDTA; antifreezes such as propylene glycol; solvents such as ethanol and isopropanol; preservatives and anti-oxidants, such as Germaben II (Sutton Laboratories); anti-dandruff agents such as zinc pyrithione and Climbazole™ (see U.S. Patent No. 4,867,971); colorants and perfume. Water, employed to make the aqueous medium but which may be present not only in liquid preparations but also in gels, pastes and cremes, is preferably filtered, irradiated and deionized water of essentially zero hardness, but it may also be tap water, although it is preferred to keep the hardness below 50 ppm, as calcium carbonate. However, other tap waters of hardnesses as high as 200 ppm will sometimes also be useful, but usually they should be avoided.

Upon rinsing the shampoo composition of the invention from the hair, the latter is found to be highly conditioned and manageable, i.e., it is easy to comb wet and feels coated but conditioned.

Upon drying the rinsed hair in a desired configuration, either by allowing it to dry under ambient conditions or by blow-drying with heated air, the dried hair will possess a high degree of style control, body and set and curl retention.

All percentages of components expressed herein, unless other indicated, are by weight, based on the weight of the composition in which the component is present.

The invention is illustrated by the following non-limiting examples wherein the following procedure was employed to prepare the shampoo formulations described herein.

### Example 1

Deionized water was charged to a main mixing vessel. While stirring, hydroxyethylcellulose (HEC) was slowly sprinkled into the vessel, followed by the addition of the cationic polymer. Heating of the mixture was initiated while continuing to mix until complete dissolution was obtained and a clear gel resulted.

The betaine, sodium cumene sulfonate (SCS) and sodium phosphate, in that order, were added to the vessel while mixing. The Polymer JR was added and the mixture heated to a first temperature of 85°C, at which point the ammonium lauryl sulfate was added with mixing.

All oil phase ingredients were added to a separate vessel and the mixture heated to 85°C.

The oil phase was added to the main mixing vessel with mixing, taking care not to promote foam formation.

Heating was discontinued and mixing was continued for at least fifteen minutes. After the mixture had cooled to 52°C, perfume was added. At 39°C, Germaben II and color were added to produce the final composition.

The formulations set forth in Table I were evaluated for style control or hold (stiffness). This stiffness test is only one aspect of style control, but was very useful for all of these formulations except for the highest conditioning compositions.

After rinsing the tresses, excess water was squeezed from the hair by squeezing the tress between the thumb and forefinger, moving the hand down the length of the tress. It was then allowed to dry overnight and the stiffness was compared to a set of standards prepared where 0 was from a normal shampoo and the tress was full and fluffy and 5 was from a system that glued each hair to adjacent hairs and appeared as a stiff rod of hair. The intermediate values showed varying intermediate levels of interfiber adhesion.

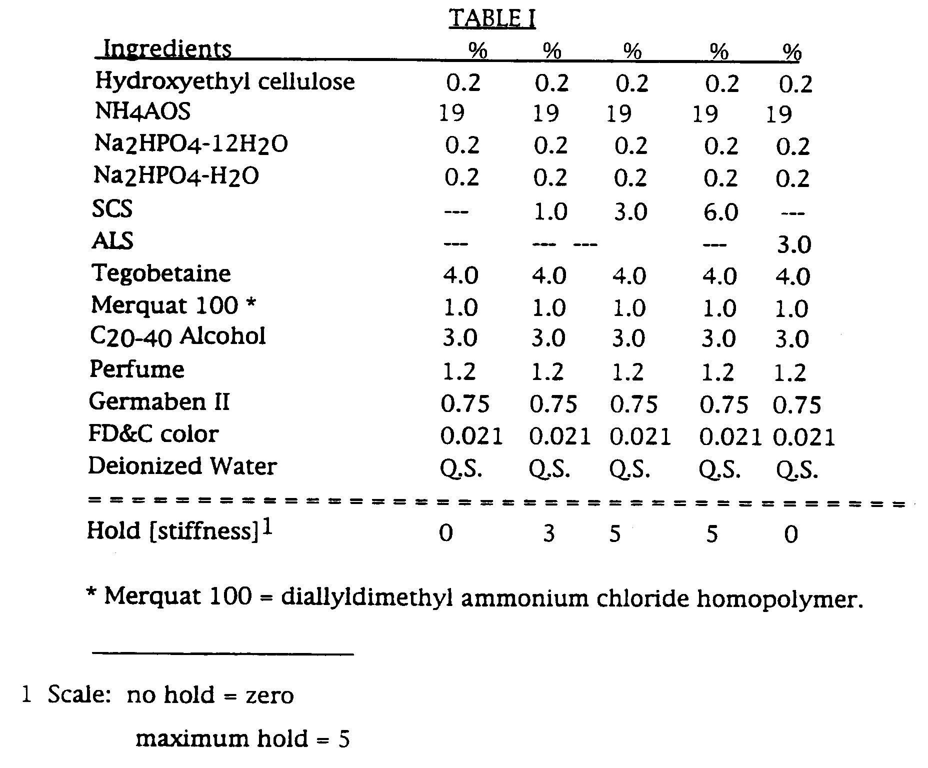

### Example 2

The procedure of Example 1 was followed to produce the formulation set forth in Table II. The formulation was tested for style control or hold (stiffness).

**TABLE II**

| Ingredients | % |
|---|---|
| Deionized Water | Q.S. |
| Merquat 100 | 1.60 |
| Betaine L-7 | 3.20 |
| Sodium Cumene Sulfonate | 4.00 |
| Sodium Phosphate M.B. | 0.16 |
| Polymer JR-30 M | 0.40 |
| Deionized Water | Q.S. |
| Ammonium Lauryl Sulfate | 14.50 |
| Unilin 425 | 2.50 |
| TA-100 | 0.20 |
| Mineral Oil D-50 | 0.40 |
| Perfume Fancy Mod. 1 | 0.50 |
| Germaben II | 0.50 |
| Color | Q.S. |

- Hold:: high
- Conditioning:: high
- Foam:: high
- Stability:: good
- Viscosity:: 4,000 cps
- pH:: 6.3 [same results with and without perfume]

### Example 3

The procedure of Example 1 was followed to prepare the formulations set forth in Table III. These formulations were tested for style control and hold (stiffness). A combing (conditioning) test was also conducted with the compositions of Table III by specially trained evaluators. A good score of 10 in the combing results would be equivalent to the results obtained using a separate shampoo and conditioner. A poor score of 0 would be equivalent to the results obtained using only a 10% sodium laureth-2 sulfate shampoo. [In Table III, Merquat 50/50 is a copolymer of 50% dimethyldiallyl ammonium chloride and 50% acrylic acid; Merquat 80/20 is a copolymer of 80% dimethyldiallyl ammonium chloride and 20% acrylamide, and Luviquat 905 is a vinyl imidazole/vinyl pyrrolidone copolymer containing at least 50% vinyl imidazole.]

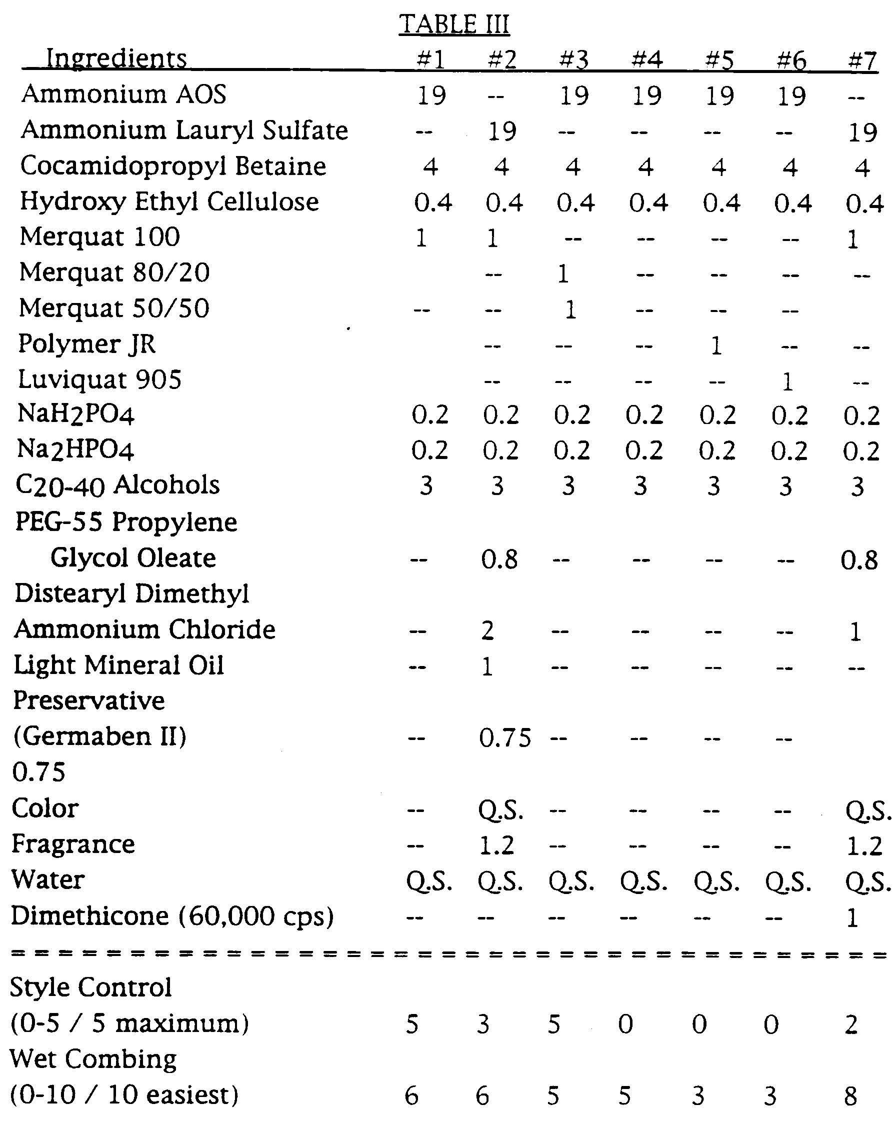

### Example 4

The procedure of Example 1 was followed to prepare the formulations set forth in Table IV. The formulations were tested for conditioning and style control. The results are set forth in Table IV below.

**TABLE IV**

| Ingredients | % | % |
|---|---|---|
| Deionized Water | Q.S. | Q.S. |
| Merquat 100 | 1.6 | 0.2 |
| Luviquat 905 | --- | 0.75 |
| Polymer JR-30M | 0.4 | 0.4 |
| Betaine L-7 | 3.2 | 2.0 |
| Sodium Cumene Sulfonate | 1.6 | 2.0 |
| Propylene Glycol | --- | 0.5 |
| Cocodiethanolamide | --- | 2.0 |
| Ammonium Lauryl Sulfate | 14.5 | 18.0 |
| Distearyl Dimonium Chloride | 0.2 | 0.5 |
| Mineral Oil D-50 | 0.4 | --- |
| Sodium Phosphate Mono-Basic | 0.16 | 0.4 |
| Unilin 425 | 2.5 | 3.0 |
| Perfume | 0.5 | 0.5 |
| Preservative | 0.5 | 0.5 |
| Color | Q.S. | Q.S |
| Dimethicone 60,000 cps | --- | 4.0 |
| Conditioning | 6 | 10 |
| Stiffness | 5 | 1 |

### Example 5

The formulations tabulated below were made as in Example 1 with varying levels of Merquat 100 and rated for style control by evaluating tress stiffness on a scale of 0-5.

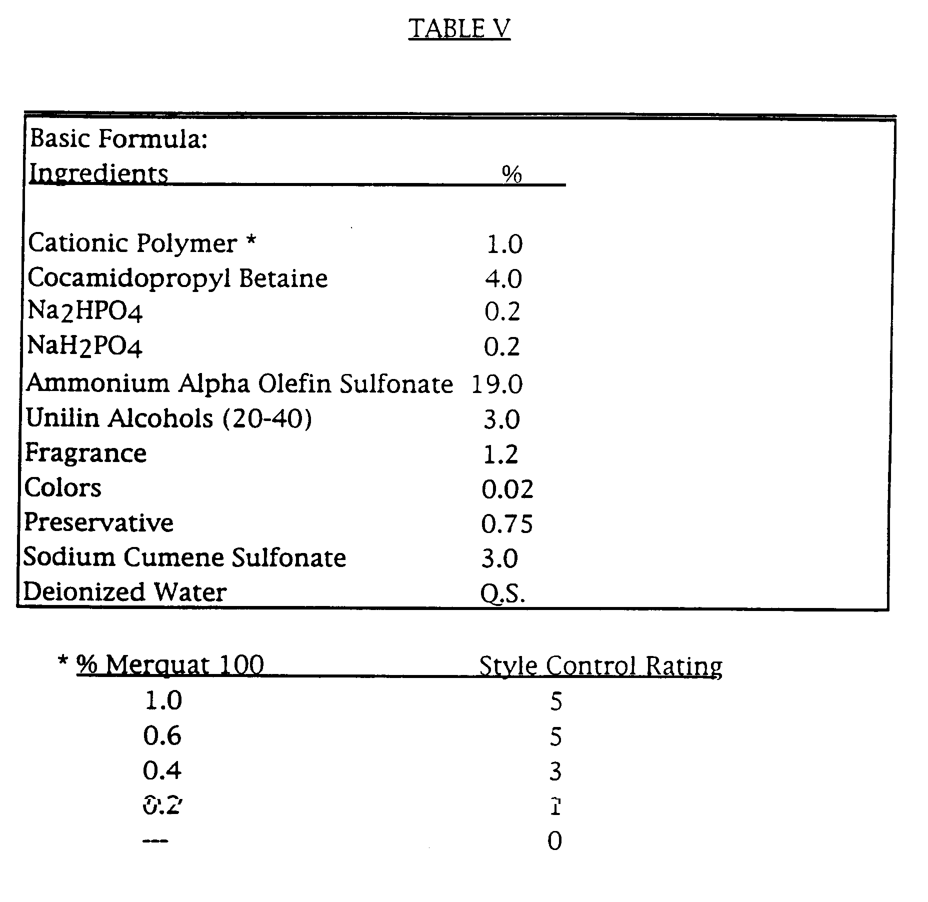

## Claims

1. A hair conditioning style control shampoo in aqueous emulsion or suspension form comprising:
a. from 0.1% to 5% of at least one cationic polymer having a hair conditioning effect and a charge density greater than about 200;
b. from 3% to 30% of a first long chain non-aromatic anionic surfactant, wherein said non-aromatic surfactant is selected from the group consisting of alkyl sulfates, alkyl ether sulfates, α-olefin sulfonates, paraffin sulfonates, ethoxylated and propoxylated alkyl ether sulfates containing 1 to 10 ethylene oxide or propylene oxide units per molecule and mixtures thereof, wherein said alkyl groups of said sulfates contain 10 to 18 carbon atoms and may be unsaturated and wherein said alkyl groups of said sulfonates contain 12 to 18 carbon atoms and may be unsaturated;
c. from 0.1% to 10% of at least one second anionic surfactact selected from the group consisting of aromatic sulfonates containing fewer than 8 alkyl carbon atoms, alkyl or alkyl ether sulfates containing up to 8 carbon atoms in the alkyl chain, alkyl sulfonates containing up to 10 carbon atoms in the alkyl group and mixtures thereof; and
d. the remainder water.

2. A hair conditioning style control shampoo according to claim 1 wherein said cationic polymer has a charge density greater than about 130.

3. A hair conditioning style control shampoo according to claim 1 additionally containing a dispersing agent which functions to stabilize the emulsion or suspension.

4. A hair conditioning style control shampoo according to claim 3 additionally containing a water-insoluble hair conditioning agent in an amount less than about 5%.

5. A hair conditioning style control shampoo according to claim 4 wherein said water-insoluble hair conditioning agent is selected from the group consisting of silicones, aminosilicones, polyalkylenes and oxidized derivatives thereof, mineral oils, paraffins, petrolatums, microcrystalline waxes, C₁₈₋₃₆ (mixed) fatty acids and triglycerides thereof and mixtures thereof, long chain amines and long chain monofunctional cationics.

6. A hair conditioning style control shampoo according to claim 1 additionally containing shampoo adjuvants.

7. A hair conditioning style control shampoo according to claim 1 wherein said cationic polymer is selected from the group consisting of dialkyldiallyl ammonium homopolymer or copolymer, a copolymer of vinyl imidazole, a copolymer of vinyl imidazole and vinyl pyrrolidone or a polymer of polyethyleneimine.

8. A hair conditioning style control shampoo according to claim 1 wherein said cationic polymer is present in an amount of from 0.1% to 5%.

9. A hair conditioning style control shampoo according to claim 1 wherein said cationic polymer is present in an amount of from 0.25% to 2.5%.

10. A hair conditioning style control shampoo according to claim 1 wherein said non-aromatic surfactant is present in an amount of from 10% to 20%.

11. A hair conditioning style control shampoo according to claim 1 wherein component (c) is an alkyl aromatic sulfonate selected from the group consisting of cumene sulfonate, xylene sulfonate, benzene sulfonate, toluene sulfonate, 1,2-benzene disulfonic acid disodium salt, 1,3-benzene disulfonic acid disodium salt or mixtures thereof.

12. A hair conditioning style control shampoo according to claim 11 wherein said aromatic surfactant is present in an amount of from 1% to 5%.

13. A hair conditioning style control shampoo according to claim 3 wherein said dispersing agent is a long chain saturated primary aliphatic alcohol or a deriative thereof having an average of 24 to 45 carbon atoms in said chain.

14. A hair conditioning style control shampoo accoridng to claim 3 wherein said dispersing agent is a long chain acylated compound selected from the group consisting of esters, acids or amides with at least 18 carbon atoms consisting of C₁₈₋₃₆ (mixed) fatty acids and triglycerides thereof, beeswax, and mixtures thereof.

15. A hair conditioning style control shampoo according to claim 3 wherein said dispersing agent is a cross-linked anionic synthetic polymer.

16. A hair conditioning style control shampoo according to claim 3 wherein said dispersing agent is a polysaccharide or a quaternized derivative thereof.

17. A hair conditioning style control shampoo according to claim 3 wherein said dispersing agent functions to render said emulsion or dispersion pearlescent.

18. A method for cleansing, conditioning and providing style control to hair with a single composition comprising shampooing the hair with a composition according to claim 1.

19. A method according to claim 18 including the additional step of rinsing the hair with water following the step of shampooing.

20. A method according to claim 19 including the additional step of drying the rinsed hair in a desired configuration to provide style control thereto.

21. A hair conditioning style control shampoo according to claim 1 wherein component (c) is a short chain aliphatic sulfate selected from the group consisting of sodium hexyl sulfate and sodium octyl sulfate.

22. A hair conditioning style control shampoo according to claim 1 wherein component (c) is a short chain aliphatic sulfonate selected from the group consisting of sodium octyl sulfonate and sodium decyl sulfonate and mixtures thereof.

23. The hair conditioning style control shampoo of claim 1 wherein said long chain non-aromatic surfactant is an α-olefin sulfonate.

24. The hair conditioning style control shampoo of claim 23 wherein said cationic polymer has a charge density greater than about 130.

25. The hair conditioning style control shampoo of claim 1 wherein said second anionic surfactant is cumene sulfonate.

26. The hair conditioning style control shampoo of claim 22 wherein said second anionic surfactant is cumene sulfonate.

## Patentansprüche

1. Haarkonditionierendes stilkontrollierendes Schampon in Form einer wäßrigen Emulsions oder Suspension, das umfaßt:
a. 0,1 % bis 5 % mindestens eines kationischen Polymers mit einem haarkonditionierenden Effekt und einer Ladungsdichte größer als etwa 200,
b. 3 bis 30 % eines ersten langkettigen nicht-aromatischen anionischen Tensids, wobei das nicht-aromatische Tensid ausgewählt ist aus der Gruppe bestehend aus Alkylsulfaten, Alkylethersulfaten, α-Olefinsulfonaten, Paraffinsulfonaten, ethoxylierten und propoxylierten Alkylethersulfaten mit 1 bis 10 Ethylenoxid- oder Propylenoxideinheiten pro Molekül und Mischungen derselben, wobei die Alkylgruppen der Sulfate 10 bis 18 Kohlenstoffatome enthalten und ungesättigt sein können und wobei die Alkylgruppen der Sulfonate 12 bis 18 Kohlenstoffatome enthalten und ungesättigt sein können,
c. 0,1 % bis 10 % mindestens eines zweiten anionischen Tensids ausgewählt aus der Gruppe bestehend aus aromatischen Sulfonaten mit weniger als 8 Alkylkohlenstoffatomen, Alkyl- oder Alkylethersulfonaten mit bis zu 8 Kohlenstoffatomen in der Alkylkette, Alkylsulfonaten mit bis zu 10 Kohlenstoffatomen in der Alkylgruppe und Mischungen derselben, und
d. als Rest Wasser.

2. Haarkonditionierendes stilkontrollierendes Schampon nach Anspruch 1, bei dem das kationische Polymer eine Ladungsdichte größer als etwa 130 aufweist.

3. Haarkonditionierendes stilkontrollierendes Schampon nach Anspruch 1, das zusätzlich ein Dispergiermittel enthält, das die Emulsion oder Suspension stabilisiert.

4. Haarkonditionierendes stilkontrollierendes Schampon nach Anspruch 3, das außerdem ein in Wasser unlösliches haarkonditionierendes Mittel in einer Menge kleiner als etwa 5 % enthält.

5. Haarkonditionierendes stilkontrollierendes Schampon nach Anspruch 4, bei dem das in Wasser unlösliche haarkonditionierende Mittel ausgewählt ist aus der Gruppe bestehend aus Silikonen, Aminosilikonen, Polyalkylenen und oxidierten Derivaten derselben, Mineralölen, Paraffinen, Vaselinen, mikrokristallinen Wachsen, (gemischten) C₁₈₋₃₆-Fettsäuren und Triglyceriden derselben sowie Mischungen derselben, langkettigen Aminen und langkettigen monofunktionellen kationischen Verbindungen.

6. Haarkonditionierendes stilkontrollierendes Schampon nach Anspruch 1, das außerdem Schamponhilfsmittel enthält.

7. Haarkonditionierendes stilkontrollierendes Schampon nach Anspruch 1, bei dem das kationische Polymer ausgewählt ist aus der Gruppe bestehend aus Dialkyldiallylammoniumhomopolymer oder -copolymer, einem Copolymer von Vinylimidazol, einem Copolymer von Vinylimidazol und Vinylpyrrolidon oder ein Polymer von Polyethylenimin.

8. Haarkonditionierendes stilkontrollierendes Schampon nach Anspruch 1, bei dem das kationische Polymer in einer Menge von 0,1 bis 5 % vorhanden ist.

9. Haarkonditionierendes stilkontrollierendes Schampon nach Anspruch 1, bei dem das kationische Polymer in einer Menge von 0,25 % bis 2,5 % vorhanden ist.

10. Haarkonditionierendes stilkontrollierendes Schampon nach Anspruch 1, bei dem das nicht-aromatische Tensid in einer Menge von 10 % bis 20 % vorhanden ist.

11. Haarkonditionierendes stilkontrollierendes Schampon nach Anspruch 1, bei dem Komponente (c) ein alkylaromatisches Sulfonat ausgewählt aus der Gruppe bestehend aus Cumolsulfonat, Xylolsulfonat, Benzolsulfonat, Toluolsulfonat, 1,2-Benzoldisulfonsäuredinatriumsalz, 1,3-Benzoldisulfonsäuredinatriumsalz und Mischungen derselben.

12. Haarkonditionierendes stilkontrollierendes Schampon nach Anspruch 11, bei dem das aromatische Tensid in einer Menge von 1 % bis 5 % vorhanden ist.

13. Haarkonditionierendes stilkontrollierendes Schampon nach Anspruch 3, bei dem das Dispergiermittel ein langkettiger gesättigter primärer aliphatischer Alkohol oder ein Derivat desselben mit einem Durchschnitt von 24 bis 45 Kohlenstoffatomen in der Kette ist.

14. Haarkonditionierendes stilkontrollierendes Schampon nach Anspruch 3, bei dem das Dispergiermittel eine langkettige acylierte Verbindung ausgewählt aus der Gruppe bestehend aus Estern, Säuren oder Amiden mit mindestens 18 Kohlenstoffatomen bestehend aus (gemischten) C₁₈₋₃₆-Fettsäuren und Triglyceriden derselben, Bienenwachs und Mischungen derselben ist.

15. Haarkonditionierendes stilkontrollierendes Schampon nach Anspruch 3, bei dem das Dispergiermittel ein vernetztes anionisches synthetisches Polymer ist.

16. Haarkonditionierendes stilkontrollierendes Schampon nach Anspruch 3, bei dem das Dispergiermittel ein Polysaccharid oder ein quarternisiertes Derivat desselben ist.

17. Haarkonditionierendes stilkontrollierendes Schampon nach Anspruch 3, bei dem das Dispergiermittel die Emulsion oder Dispersion fischsilbrig macht.

18. Verfahren zur Reinigung, Konditionierung und Lieferung von Stilkontrolle bei Haar mit einer einzigen Zusammensetzung, bei dem das Haar mit einer Zusammensetzung gemäß Anspruch 1 schamponiert wird.

19. Verfahren nach Anspruch 18, das den zusätzlichen Schritt des Ausspülens des Haars mit Wasser im Anschluß an das Schamponieren umfaßt.

20. Verfahren nach Anspruch 19, das den zusätzlichen Schritt des Trocknens des gespülten Haars in einer gewünschten Konfiguration umfaßt, um ihm Stilkontrolle zu verleihen.

21. Haarkonditionierendes stilkontrollierendes Schampon nach Anspruch 1, bei dem Komponente (c) ein kurzkettiges aliphatisches Sulfat ausgewählt aus der Gruppe bestehend aus Natriumhexylsulfat und Natriumoctylsulfat ist.

22. Haarkonditionierendes stilkontrollierendes Schampon nach Anspruch 1, bei dem Komponente (c) ein kurzkettiges aliphatisches Sulfonat ausgewählt aus der Gruppe bestehend aus Natriumoctylsulfonat und Natriumdecylsulfonat sowie Mischungen derselben ist.

23. Haarkonditionierendes stilkontrollierendes Schampon nach Anspruch 1, bei dem das langkettige nicht-aromatische Tensid ein α-Olefinsulfonat ist.

24. Haarkonditionierendes stilkontrollierendes Schampon nach Anspruch 23, bei dem das kationische Polymer eine Ladungsdichte größer als etwa 130 aufweist.

25. Haarkonditionierendes stilkontrollierendes Schampon nach Anspruch 1, bei dem das zweite anionische Tensid Cumolsulfonat ist.

26. Haarkonditionierendes stilkontrollierendes Schampon nach Anspruch 22, bei dem das zweite anionische Tensid Cumolsulfonat ist.

## Revendications

1. Shampooing donnant de la tenue de coiffure, conditionnant les cheveux, sous forme d'émulsion ou de suspension aqueuse, comprenant :
a. 0,1 % à 5 % d'au moins un polymère cationique ayant un effet de conditionnement capillaire et une densité de charge supérieure à environ 200 ;
b. 3 % à 30 % d'un premier agent tensio-actif anionique non aromatique à longue chaîne, ledit agent tensio-actif non aromatique étant choisi dans le groupe formé par les alkylsulfates, les alkyléthersulfates, les α-oléfinesulfonates, les paraffinesulfonates, les alkyléthersulfates éthoxylés et propoxylés contenant 1 à 10 motifs oxyde d'éthylène ou oxyde de propylène par molécule et leurs mélanges, où lesdits groupes alkyle desdits sulfates contiennent 10 à 18 atomes de carbone et peuvent être insaturés et où lesdits groupes alkyle desdits sulfonates contiennent 12 à 18 atomes de carbone et peuvent être insaturés ;
c. 0,1 % à 10 % d'au moins un second agent tensio-actif anionique choisi dans le groupe formé par les sulfonates aromatiques contenant moins de 8 atomes de carbone alkyliques, les alkyl- ou alkyléther-sulfates contenant jusqu'à 8 atomes de carbone dans la chaîne alkyle, les alkylsulfonates contenant jusqu'à 10 atomes de carbone dans le groupe alkyle et leurs mélanges ; et
d. le reste d'eau.

2. Shampooing donnant de la tenue de coiffure, conditionnant les cheveux selon la revendication 1, dans lequel ledit polymère cationique a une densité de charge supérieure à environ 130.

3. Shampooing donnant de la tenue de coiffure, conditionnant les cheveux selon la revendication 1, contenant de plus un agent dispersant qui agit pour stabiliser l'émulsion ou suspension.

4. Shampooing donnant de la tenue de coiffure, conditionnant les cheveux selon la revendication 3, contenant de plus un agent conditionnant pour cheveux insoluble dans l'eau en une quantité inférieure à environ 5 %.

5. Shampooing donnant de la tenue de coiffure, conditionnant les cheveux selon la revendication 4, dans lequel ledit agent conditionnant pour cheveux insoluble dans l'eau est choisi dans le groupe formé par les silicones, les aminosilicones, les polyalkylènes et leurs dérivés oxydés, les huiles minérales, les paraffines, les pétrolatums, les cires microcristallines, les acides gras en c₁₈-c₃₆ (mixtes) et leurs triglycérides et leurs mélanges, les amines à longue chaîne et les composés cationiques monofonctionnels à longue chaîne.

6. Shampooing donnant de la tenue de coiffure, conditionnant les cheveux selon la revendication 1, contenant de plus des adjuvants de shampooing.

7. Shampooing donnant de la tenue de coiffure, conditionnant les cheveux selon la revendication 1, dans lequel ledit polymère cationique est choisi dans le groupe formé par un homopolymère ou copolymère de dialkyldiallylammonium, un copolymère de vinylimidazole, un copolymère de vinylimidazole et de vinylpyrrolidone ou un polymère de polyéthylèneimine.

8. Shampooing donnant de la tenue de coiffure, conditionnant les cheveux selon la revendication 1, dans lequel ledit polymère cationique est présent en une quantité de 0,1 % à 5 %.

9. Shampooing donnant de la tenue de coiffure, conditionnant les cheveux selon la revendication 1, dans lequel ledit polymère cationique est présent en une quantité de 0,25 % à 2,5 %.

10. Shampooing donnant de la tenue de coiffure, conditionnant les cheveux selon la revendication 1, dans lequel ledit agent tensio-actif non aromatique est présent en une quantité de 10 % à 20 %.

11. Shampooing donnant de la tenue de coiffure, conditionnant les cheveux selon la revendication 1, dans lequel le composant (c) est un sulfonate alkylaromatique choisi dans le groupe formé par un cumènesulfonate, un xylènesulfonate, un benzènesulfonate, un toluènesulfonate, le sel disodique d'acide 1,2-benzènedisulfonique, le sel disodique d'acide 1,3-benzènedisulfonique ou un mélange d'entre eux.

12. Shampooing donnant de la tenue de coiffure, conditionnant les cheveux selon la revendication 11, dans lequel ledit agent tensio-actif aromatique est présent en une quantité de 1 % à 5 %.

13. Shampooing donnant de la tenue de coiffure, conditionnant les cheveux selon la revendication 3, dans lequel ledit agent dispersant est un alcool aliphatique primaire saturé à longue chaîne ou un dérivé de celui-ci, ayant en moyenne 24 à 45 atomes de carbone dans ladite chaîne.

14. Shampooing donnant de la tenue de coiffure, conditionnant les cheveux selon la revendication 3, dans lequel ledit agent dispersant est un composé acylé à longue chaîne choisi dans le groupe formé par tes esters, acides ou amides ayant au moins 18 atomes de carbone consistant en acides gras en C₁₈-C₃₆ (mixtes) et leurs triglycérides, la cire d'abeilles, et leurs mélanges.

15. Shampooing donnant de la tenue de coiffure, conditionnant les cheveux selon la revendication 3, dans lequel ledit agent dispersant est un polymère synthétique anionique réticulé.

16. Shampooing donnant de la tenue de coiffure, conditionnant les cheveux selon la revendication 3, dans lequel ledit agent dispersant est un polysaccharide ou un dérivé quaternisé de celui-ci.

17. Shampooing donnant de la tenue de coiffure, conditionnant les cheveux selon la revendication 3, dans lequel ledit agent dispersant a pour fonction de rendre nacrée ladite émulsion ou dispersion.

18. Procédé pour laver, conditionner et donner de la tenue de coiffure à des cheveux avec une seule composition, comprenant un shampooing des cheveux avec une composition selon la revendication 1.

19. Procédé selon la revendication 18, comprenant l'étape supplémentaire de rinçage des cheveux avec de l'eau après l'étape de shampooing.

20. Procédé selon la revendication 19, comprenant l'étape supplémentaire de séchage des cheveux rincés dans une configuration désirée pour leur conférer de la tenue de coiffure.

21. Shampooing donnant de la tenue de coiffure, conditionnant les cheveux selon la revendication 1, dans lequel le composant (c) est un sulfate aliphatique à chaîne courte choisi dans le groupe formé par l'hexylsulfate de sodium et l'octylsulfate de sodium.

22. Shampooing donnant de la tenue de coiffure, conditionnant les cheveux selon la revendication 1, dans lequel le composant (c) est un sulfonate aliphatique à chaîne courte choisi dans le groupe formé par l'octylsulfonate de sodium et le décylsulfonate de sodium et leurs mélanges.

23. Shampooing donnant de la tenue de coiffure, conditionnant les cheveux de la revendication 1 , dans lequel l'agent tensio-actif non aromatique à longue chaîne est un α-oléfinesulfonate.

24. Shampooing donnant de la tenue de coiffure, conditionnant les cheveux de la revendication 23, dans lequel ledit polymère cationique à une densité de charge supérieure à environ 130.

25. Shampooing donnant de la tenue de coiffure, conditionnant les cheveux de la revendication 1, dans lequel ledit second agent tensio-actif est un cumènesulfonate.

26. Shampooing donnant de la tenue de coiffure, conditionnant les cheveux de la revendication 22, dans lequel ledit second agent tensio-actif est un cumènesulfonate.
